# EUROPEAN PATENT SPECIFICATION

(11) **EP 1 289 414 B1**
(45) Date of publication and mention of the grant of the patent: **14.09.2005**
(21) Application number: 01925829.2
(22) Date of filing: 18.04.2001
(51) Int. Cl.: A61B 5/05, A61B 6/00

(54) **COMBINED IMPEDANCE IMAGING AND MAMMOGRAPHY**
KOMBINIERTE IMPEDANZBILDGEBUNG UND MAMMOGRAPHIE
IMAGERIE DE L'IMPEDANCE ET MAMMOGRAPHIE COMBINEES

(30) Priority: 21.05.2000 WO PCT/IL00/00287; 14.12.2000 WO PCT/IL00/00839
(43) Date of publication of application: 12.03.2003
(73) Proprietor: Transscan Medical Ltd, 10550 Migdal Haemek (IL)
(72) Inventor: STEINBERG, Sebastian, 27043 Kiryat Bialik (IL); WIMISNER, Yoav, 30900 Zichron Yaakov (IL); NACHALIEL, Ehud, 15295 Lower Galilee (IL); MALONEK, Dov, 36000 Kiryat-Tivon (IL)
(74) Representative: van Westenbrugge, Andries
(86) International application number: PCT/IL2001/000359
(87) International publication number: WO 2001/089380

(56) References cited:
- EP-A- 0 945 103
- WO-A-99/12470
- US-A- 4 291 708
- US-A- 4 763 660
- US-A- 5 810 742
- FRERICHS I ET AL.: "Electrical impedance tomography in monitoring experimental lung injury" INTENSIVE CARE MEDICINE, vol. 24, 1998, pages 829-836, XP002180964

## Description

### FIELD OF THE INVENTION

The present invention relates generally to medical imaging techniques. In particular the present invention relates to performing impedance imaging in conjunction with mammography.

### BACKGROUND OF THE INVENTION

Early detection of cancerous tumors increases the chances of success in curing breast cancer. Several methods are known to screen the breast in order detect suspicious tumors. Two of these methods are X-ray mammography and impedance imaging, described, for example, in U.S. patent 4,291,708 to Frei and in U.S. patent 5,810,742 to Pearlman. In impedance imaging, a multi-element impedance probe is placed on a breast of a patient and one or more electrodes apply electrical signals to the subject. Each of the elements of the probe senses electrical signals from the breast and accordingly an image of the breast is generated. The multi-element probe comprises a plurality of conductive elements.

U.S. patent 6,157,697 describes the use of an apparatus for examining a breast by means of X-rays and by electric impedance measurement. The apparatus can be used to generate both X-ray and impedance images of the breast. A fusion image may be formed from a combination of the images. In order to allow easy comparison and fusion of the images, the images are taken during a single session while the breast is in the same position, and from substantially the same direction. That is, the X-ray images are taken while electrodes used for generating an impedance image are positioned on the breast.

The '697 patent suggests using an impedance imaging probe which is formed of three layers. A first layer comprises a plurality of electrodes and lead wires, which electrodes are used in the impedance imaging. Although the electrodes are suggested as comprising a material which attenuates X-ray poorly, the difference between the X-ray intensity passing through the electrodes and the intensity passing through areas between the electrodes may cause artifacts on the x-ray images taken while the impedance probe is in place. Therefore, the '697 patent suggests using a second layer, separated from the electrodes by an insulation (third) layer, which comprises the electrode materials in a mirror image to the first layer. The additional layer is not used in any way for the impedance imaging and is provided in order to prevent formation of shadow artifacts on the x-ray images.

### SUMMARY OF THE INVENTION

An aspect of some embodiments of the present invention relates to a multi-element impedance probe which includes conductive materials in one or more layers. At least one of the layers includes an isolating material, which has an x-ray absorption level substantially equal to that of the conductive materials, between the conductive materials of the layer. The isolating material is optionally included throughout an imaging region of the probe. In some embodiments of the invention, the isolating material has substantially the same x-ray absorbency as the conductive niaterial of the same layer. Alternatively, the isolating material of a layer has a different thickness than the conductive material of the layer, such that the x-ray absorption of the conductive and isolating materials of the layer is substantially equal. Further alternatively, the isolating material comprises a mixture of materials which has substantially the same x-ray absorption as the conductive material.

In some embodiments of the invention, the impedance probe includes a plurality of layers including, for example, a body contact layer, one or more wire leading layers, a substrate layer and one or more isolating layers. It is noted that the wire leading layers and body contact layer include both conductive materials and the isolating materials between them. In some embodiments of the invention, the conductive and non-conductive materials of a single layer have substantially the same heigbt. In other embodiments of the invention, the substantive conductive and non-conductive materials of a single layer have different heights. The difference in the heights may be left empty (e.g., in the body contact layer), or may be filled in with low x-ray absorbent materials (e.g., low x-ray absorbency plastics) for example of an adjacent isolating layer. Optionally, each of the layers has substantially the same x-ray absorption over its entire surface area within the imaging region. Having each of the layers of the multi-element probe have equal absorption levels over the surface area included in the imaging region prevents formation of artifacts in images taken in an angle relative to the impedance probe. In addition, using an isolating material for equalizing the x-ray absorption level of the layers, allows simple use of a plurality of conductive layers in a single probe.

An aspect of some embodiments of the invention relates to a multi-element impedance probe in which the x-ray absorption level of the non-conductive layers of surface regions of the probe are a function of the x-ray absorption level of the conductive layers of the surface regions. In some embodiments of the invention, different surface regions of the probe have substantially different x-ray absorption levels of their non-conductive layers. Optionally, the probe has a substantially equal x-ray absorption level, substantially over its entire imaging region. Optionally, the probe does not include non-functional conductive portions.

In some embodiments of the invention, the conductive elements of the impedance probe and the insulation separating the elements are formed of materials which have similar x-ray absorbency. Optionally, the materials are substantially x-ray transparent materials, which have a very low X-ray absorbency. In an exemplary embodiment of the invention, the conductive elements comprise aluminum and the insulation comprises Alumina (i.e., Al₂O₃) and/or silicone.

Alternatively or additionally, the thickness of the probe at different areas is varied according to the X-ray attenuation of the materials in each specific surface area. Optionally, the conductive elements are held by a substrate which has a variable thickness according to the size and shape of the conductive elements.
There is thus provided, in accordance with an exemplary embodiment of the invention a probe for impedance imaging comprising:
a plurality of conductive elements adapted to contact tissue of a subject;
a plurality of conductive wires connecting the conductive elements to an external connector; and
one or more non-conductive materials,
wherein the x-ray absorbance of the non-conductive materials, in at least some of the surface areas of the probe, is a function of the x-ray absorbance of the conductive wires and elements in the same surface area, such that the probe has substantially the same x-ray absorbance over most of the surface area of the probe.

In an embodiment of the invention, the one or more non-conductive materials comprise a substrate layer having different thickness at different surface areas of the probe. Optionally, the substrate layer is thinner in surface areas of the probe which include conductive wires.

In an embodiment of the invention, the one or more non-conductive materials comprise at least one isolating material which has an x-ray absorbency substantially equal to the x-ray absorbency of the conductive elements or of the conductive wires. Optionally, the plurality of conductive elements comprise an aluminum-based deposit and the at least one isolating material comprises alumina.

In an embodiment of the invention, the one or more non-conductive materials comprise at least one isolating material which is deposited between at least some of the conductive elements.

In an embodiment of the invention, the x-ray absorption level of at least some of the surface areas including the conductive elements is substantially equal to the absorption level of at least some of the surface areas not including the conductive elements.

Optionally, the plurality of lead wires are included in a single layer which has a substantially equal x-ray absorption level over substantially its entire surface area.

Optionally, the probe does not include conductive portions which are not included in an electrical path between one of the conductive elements and the external connector.

Optionally, the probe has an x-ray absorption level of less than 6%.

### BRIEF DESCRIPTION OF THE DRAWINGS

Exemplary non-limiting embodiments of the invention will be described with reference to the following description of embodiments in conjunction with the figures. Identical structures, elements or parts which appear in more than one figure are preferably labeled with a same or similar number in all the figures in which they appear, in which:
Fig. 1 is a schematic illustration of a dual-purpose apparatus for mammography and impedance-imaging, in accordance with an embodiment of the present invention;
Fig. 2 is a schematic illustration of an impedance probe and a probe housing, in accordance with an embodiment of the present invention;
Figs. 3A and 3B are schematic views of the impedance probe of Fig. 2, in accordance with an embodiment of the present invention;
Fig. 4 is a schematic cross-section view of a portion of an impedance probe, in accordance with an embodiment of the present invention; and
Fig. 5 is a flowchart of the acts performed in a dual modality imaging procedure, in accordance with an embodiment of the present invention.

### DETAILED DESCRIPTION OF PREFERRED EMBODIMENTS

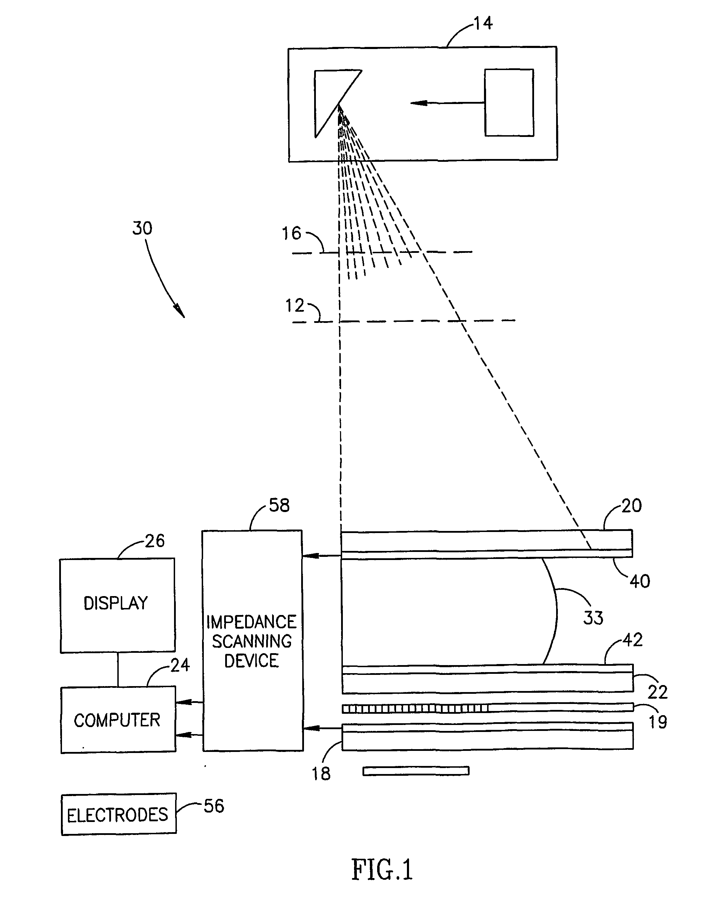

Fig. 1 is a schematic illustration of a dual-purpose apparatus 30 for mammography and impedance-imaging, in accordance with an embodiment of the present invention. Dual-purpose apparatus 30 comprises an x-ray mammogram of any type known in the art, of which Fig. 1 shows an x-ray tube 14, an image receptor 18 (which may be analog or digital), filters 16, a collimator 12 and an anti-scattering grid 19. In addition, Fig. 1 shows a compression plate 20 and a support plate 22 which are adapted to receive an examined breast 33. For example, the mammogram may comprise a LORAD® M-IV mammogram (manufactured by LORAD, a subsidiary of Trex Medical Corporation, Danbury, CT) which includes a cellular grid system for contrast and visual enhancement. Alternatively or additionally, the mammogram may have stereoscopic capability for obtaining x-ray images from at least two viewing angles. The mammogram may be upright for use with sitting or standing patients. Optionally, the mammogram can be rotated along the horizontal axis so as to obtain x-ray measurements at any angle. Alternatively, the mammogram may have fewer degrees of freedom. Further alternatively, the mammogram is horizontal for use with prone patients. It should be noted that in general, any mammogram may be fitted in accordance with the present invention, including mammograms designed and/or manufactured without intention to be used in accordance with the present invention.

One or more multi-element impedance probes (in Fig. 1, probes 40 and 42) for sensing impedance signals, and/or for applying signals, are placed on breast 33. An electrode 56 for applying electrical stimulation signals is positioned on a surface of the subject whose breast 33 is examined. Optionally, electrode 56 is placed remote from the breast or otherwise not in the path of the x-ray beams from x-ray tube 14. Alternatively, electrode 56 is placed on the breast, for example on the nipple. Optionally, electrode 56 comprises x-ray translucent materials, for example, a single thin aluminum electrode. Alternatively, electrode 56 comprises a multi-element probe, for example having a similar structure and/or according to the principles described above with relation to probe 40.

Optionally, a computer 24 receives the sensed signals from probe 40, from probe 42 and/or from image receptor 18 (when a digital x-ray detector is used, for example), and provides an indication of the state of breast 33. For example, computer 24 may display an image of breast 33 on a monitor 26.

In some embodiments of the invention, apparatus 30 comprises an electrical impedance scanning device 58 which controls the sensing of the impedance signals and the applying of electrification signals to the patient. Scanning device 58 may be substantially any suitable electrical impedance scanning device known in the art, for example, a T-Scan™ 2000 Impedance Scanner of TransScan, Israel, or as described in US patents 5,810,742, 4,458,694, PCT publication PCT/WO01/64102, filed March 1, 2000, PCT application PCT/WO01/43630, filed December 14, 2000 and/or US Patent 6,560,480, filed December 14, 1999. Alternatively or additionally, some or all of the tasks of scanning device 58 are performed by computer 24.

In some embodiments of the invention, each of the elements of probes 40 and/or 42 is controlled separately such that at any single moment some of the elements may sense signals, others may provide excitation (optionally at different phases, frequencies or amplitudes), and still others may be passive. Alternatively or additionally, the elements of one or more of probes 40 and 42 are controlled in groups, for example in lines or circles including a plurality of elements. Further alternatively or additionally, the control of the elements of probes 40 and 42 is as described in any of the above referenced patent documents.

In some embodiments of the invention, apparatus 30 comprises only a single probe (e.g., 40), which is attached to compression plate 20. By placing probe 40 within the path of x-rays used in x-ray imaging of the breast, the images generated by probe 40 cover substantially the same area as the images acquired by receptor 18. When impedance probe 40 is located between X-ray tube 14 and breast 33, X-rays absorbed by probe 40 which therefore do not participate in generating an X-ray image by receptor 18, do not impinge on breast 33. Thus, probe 40 may have a certain degree of uniform x-ray absorption without adversely affecting the x-ray image or the amount of x-rays absorbed by breast 33. The x-ray intensity of the beams generated by head 14 may be adjusted responsive to the x-ray absorption of probe 40.

In other embodiments of the invention, apparatus 30 comprises a pair of probes 40 and 42 attached, respectively, to compression plate 20 and support plate 22. As described, for example, in the above mentioned U.S. patent 5,810,742 and/or in PCT publication WO01/43630, a pair of impedance probes 40 and 42 can be used in a multitude of impedance imaging procedures which provide additional information on anomalies within breast 33, such as its location (e.g., depth) and/or type. Alternatively, apparatus 30 comprises only probe 42, which is attached to support plate 22.

In some embodiments of the invention, probes 40 and/or 42 are permanently affixed to compression plate 20 and/or support plate 22. Alternatively or additionally, impedance probe 40 serves as compression plate 20 and/or probe 42 serves as support plate 22. Optionally in this alternative, impedance probes 40 and/or 42 are strengthened with a stiff plastic board, or another, preferably radiolucent, stiff material.

Alternatively or additionally, impedance probes 40 and/or 42 are detachably attached to a breast side of compression plate 20 and/or of support plate 22. Having probes 40 and/or 42 detachably connected to plates 20 and/or 22, respectively, allows performing imaging sessions with only x-rays or with only a single impedance probe 40 or 42. For example, in some cases, in order to reduce x-ray exposure, only impedance probe 40 or 42 is used. In other cases, when a high precision level is required, both impedance probes 40 and 42 are used. In addition, having probes 40 and/or 42 detachably connected to plates 20 and/or 22, respectively, allows replacement of probes 40 and/or 42 for each patient.

Impedance probes 40 and/or 42 may be affixed to their respective plates using any suitable method, such as tongue-in-grove, male-female snaps, screws, nuts and bolts, adhesives or Velcro fasteners, and/or any of the methods described in PCT publication WO 01/89379. It is noted that probes 40 and/or 42 may have the same size as the plates to which they are connected or may have different sizes than the plates.

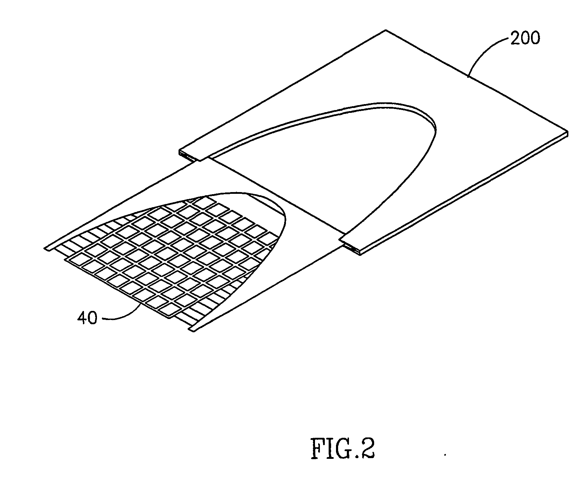

Fig. 2 is a schematic illustration of an impedance probe 40 and a probe housing 200, in accordance with an embodiment of the present invention. Probe housing 200 receives probe 40, providing mechanical support and/or electrical connection. Optionally, probe 40 is disposable, while housing 200 is used for many patients. Probe housing 200 may be connected to support plate 20 using any of the above described methods.

In some embodiments of the invention, impedance probe 40 and compression plate 20 are aligned in a precise and repeatable alignment which allows simple registration of images acquired by probe 40 with images acquired by receptor 18. In those embodiments in which impedance probe 40 is removable, the precise and repeatable alignment is optionally defined by a groove designed to receive the probe, e.g., probe housing 200, and/or the positions of the attachment devices. Alternatively or additionally, human identifiable markings, for example felt-tip markings, instructing an operator how to align compression plate 20 and probe 40 are marked on the plate and/or probe.

Alternatively or additionally, registration of the images produced by impedance probe 40 with the X-ray images, is performed each time probe 40 and plate 20 are connected and/or for each imaging procedure. In some embodiments of the invention, probe 40 comprises x-ray absorbing markings, which appear in x-ray images acquired by x-ray receptor 18, deposited thereon at predetermined positions. Optionally, the x-ray absorbing markings are not located above sensing elements of probe 40 and/or are outside the region of interest of most images. Alternatively, the absorbing markings are located on probe housing 200 or on any other device having a predetermined spatial relation with probe 40. Further alternatively, the x-ray absorbing markings are removably attached to impedance probe 40. When alignment is required they are put in place and when x-ray images are acquired they are removed. Alternatively or additionally, the imprints of the x-ray absorbing markings are removed from impedance images by image processing methods. Optionally, the absorbing markings identify the orientation of impedance probe 40, for example using a "right" and/or "left" marking.

Optionally, the absorbing markings comprise a material that is sufficiently absorbing to show on the x-ray image, but not so absorbing that it can cause serious artifacts, for example a thick layer of aluminum or a thin layer of platinum or gold. In some embodiments of the invention, the absorbing markings comprise at least two perpendicular lines which define at least one junction, optionally two junctions. In some embodiments of the invention, the absorbing markings are visible to humans. Alternatively or additionally, the location of the absorbing markings on impedance probe 40 are known by computer 24, and registration is performed accordingly.

It is noted that any of the above described attachment methods of probe 40 and compression plate 20, may be used to attach probe 42 and support plate 22.

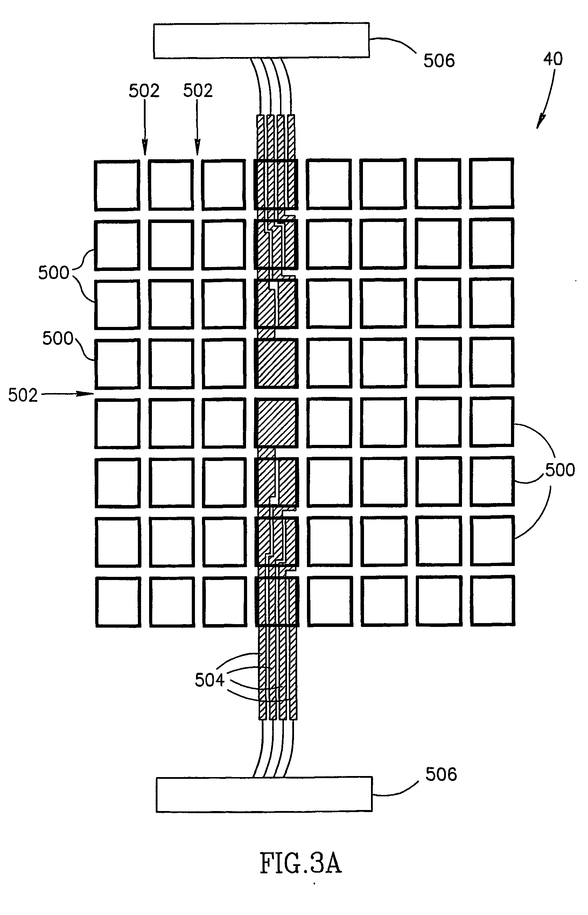

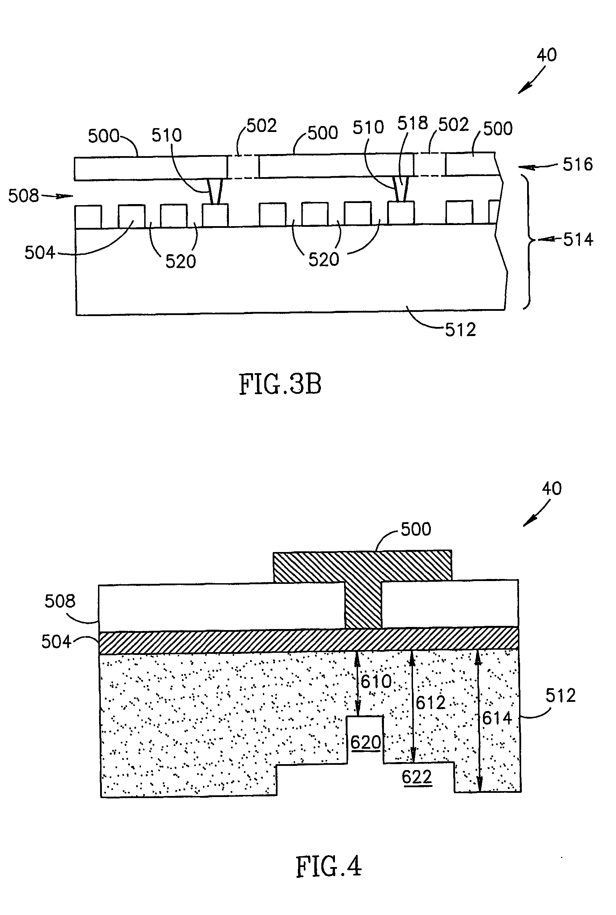

Fig. 3A is a schematic top view of impedance probe 40 shown in Fig. 2, in accordance with an embodiment of the present invention. Fig. 3A also shows lead wires 504 beneath one row of pads 500. The remaining lead wires are not shown, so as not to clutter Fig. 3A. Fig. 3B is a side cross-sectional view of the impedance probe 40 of Fig. 3A. Probe 40 comprises a plurality of electrode pads 500, for example arranged in a two dimensional array. As shown, probe 40 comprises an 8x8 array of pads 500. It is noted, however, that the present invention may be implemented with substantially any number of pads 500, in substantially any useful arrangement. In an exemplary embodiment of the invention, probe 40 comprises 45x60 pads 500, each pad having a square area of about 4x4 mm.

In some embodiments of the invention, electrode pads 500 are isolated from each other by isolating strips 502. Optionally, isolating strips 502 are as narrow as possible, such that most of the area of the top surface of probe 40 is uniform and does not cause artifacts in x-ray images acquired by receptor 18. Alternatively or additionally, isolating strips 502 have a width selected as a compromise between the desire to minimize artifacts on the x-ray images, and the need to minimize cross talk between the electrical signals of pads 500. In an exemplary embodiment of the invention, the width of insulation strips 502 is about 0.2 mm. A layer including pads 500 and isolating strips 502 is referred to herein as a contact layer 516.

Beneath electrode pads 500, probe 40 comprises, for each pad 500, a lead wire 504 which connects the pad to an external connector 506 which leads signals from pads 500 to scanning device 58 (Fig. 1). In some embodiments of the invention, external connector 506 is included in probe housing 200 (Fig. 2). An isolating layer 508 separates pads 500 from lead wires 504. In some embodiments of the invention, isolating strips 520 separate between lead wires 504. Respective vias 510 within isolating layer 508 connect each of pads 500 to its respective lead wire 504. Optionally, a substrate layer 512 beneath lead wires 504 provides probe 40 with durability and/or isolates lead wires 504 from the surroundings.

In some embodiments of the invention, electrode pads 500 and isolating strips 502 comprise materials which have substantially the same substance X-ray absorbency. Generally, the substance x-ray absorbency is a function of the atomic number Z of the material. Thus, shadows are not formed on X-ray images acquired by receptor 18, due to a difference between the X-ray absorption of different areas of probe 40.

In an exemplary embodiment of the invention, electrode pads 500 comprise aluminum (for example 0.1-0.2 µm) and isolating strips 502 comprise alumina and/or silicone. Optionally, electrode pads 500 have a very thin carbon layer (e.g., 0.1 µm) on the surface which contacts the breast. In some embodiments of the invention, isolating strips 502 include, in addition to the alumina, a respective thin layer of a plastic with similar x-ray absorbency to that of the carbon so as to compensate for the carbon. Alternatively, isolating strips 502 include only alumina, as the carbon layer is very thin. Alternatively to electrode pads 500 including aluminum, electrode pads 500 comprise a carbon, e.g., amorphous carbon, and isolating strips 502 comprise a plastic material with similar x-ray absorbency, e.g., polyester. In an exemplary embodiment of the invention, pads 500 comprise thin carbon layers of between about 0.1-20µm.

Alternatively to having the same x-ray absorbency, isolating strips 502 have a thickness which compensates for the different x-ray absorbencies, such that electrode pads 500 and isolating strips 502 have the same absorption level.

Alternatively, the gaps between electrode pads 500 are not filled in, and other methods, such as described below, are used to minimize artifact generation.

In some embodiments of the invention, lead wires 504 and the isolating strips 520 therebetween are similarly formed of materials with substantially the same X-ray absorption levels. Alternatively or additionally, strips 520 are formed of the same material as isolating layer 508. Similarly, in some embodiments of the invention, vias 510 and isolating layer 508 comprise materials with substantially the same X-ray absorption levels. Alternatively or additionally, vias 510 have a width and/or length (e.g., 10 µm) substantially smaller than the resolution of the X-ray imaging apparatus, such that the x-ray absorption of the material of vias 510 is very small relative to the amount of x-ray energy influencing a smallest resolution area on receptor 18.

In some embodiments of the invention, the x-ray absorption differences over contact layer 516 and/or over the layers including wires 504 are less than 10%, 5%, 2%, 0.5% or even 0.1%.

Substrate 512, and/or isolating layer 508 optionally comprise a radiolucent, nonconductive material such as a low x-ray absorbency plastic (for example, Mylar®, polyamide, polyimide, polyurethane, polycarbonate, or Tyvec®) or paper.

In some embodiments of the invention, electrode pads 500, lead wires 504 and/or vias 510 are formed of materials which have a relatively high ratio of their conductivity to their x-ray absorbency. Such materials may have a high conductivity and comparatively high x-ray absorbency relative to other low absorbency materials, e.g., aluminum, or may have a relatively low conductivity with a low x-ray absorbency, e.g., graphite based deposits and/or carbon layers. Alternatively, any other material with a comparative ratio between electrical conductivity and x-ray absorbency, such as amorphous carbon or a carbon slightly doped with a conductive material such as silver, may be used.

Optionally, the thickness of pads 500 and/or the cross section of lead wires 504 and/or vias 510, are chosen as a trade-off between their x-ray absorbency and their conductance. Vias 510, as they are very short, optionally have a very small cross-section, as described above. Lead wires 504 optionally have a conductance of up to about 100 Ohm in order to minimize voltage drop of the signals sensed by pads 500 as they are provided by connector 506. For low conductance materials, e.g., carbon, such conductance requires a cross section of between about 1000-3000 µm², while for high conductance materials, e.g., aluminum, such conductance requires a cross section of between about 20-80 µm².

In some embodiments of the invention, lead wires 504 have a circular and/or rectangular cross sectional shape, with a similar width and length. Thus, the dimensions of lead wires 504 are very small, optionally smaller than the resolution of receptor 18, thus decreasing the effect of the wires on the acquired x-ray images.

Alternatively or additionally, some or all of lead wires 504 comprise a very thin film with a relatively wide width. The very thin film has a very low x-ray absorption level and therefore does not substantially cause artifacts on images generated by receptor 18. The use of wide lead wires 504 spreads artifacts caused by the lead wires over a large area and therefore the artifacts are different from anomaly imprints on x-ray images. Thus, the artifacts do not substantially interfere with identifying anomaly imprints on the x-ray image. In some embodiments of the invention, lead wires 504 are included on a plurality of layers so as to allow room for wide lead wires. Further alternatively or additionally, some or all of lead wires 504 comprise very narrow, optionally relatively thick, wires which cover a very small area of probe 40.

In some embodiments of the invention, all the materials included in probe 40 are bio-compatible and bio-stable. Alternatively, the materials of contact layer 516 are bio-compatible and bio-stable while the materials of probe base 514 have lower bio-compatibility. Thus, the materials of probe base 514 may be less x-ray absorbent, lighter, and/or more conductive or isolating (as appropriate). Optionally, some or all of the materials are hypoallergenic.

In some embodiments of the invention, during manufacture, a substrate 512 is carved into shape. Thereafter, lead wires 504 are placed on substrate 512 using any available production method, such as methods, e.g., photo-resist and/or etching, used in production of semi-conductors, printed circuit boards (PCBs) and/or flex circuits. The placement of the amorphous carbon may be performed using any method known in the art, such as laser deposition and/or chemical vapor deposition. Strips 520 between lead wires 504 are then optionally filled in with an isolating material, as described above. Alternatively or additionally, lead wires 504 are covered with a uniform thickness layer of the isolating material of strips 520. In this covering, strips 520 are filled in. Lead wires 504 and strips 520 are then optionally covered by isolating layer 508.

At the points at which pads 500 are to be placed, holes are optionally drilled in isolating layer 508, in order to receive vias 510. Optionally, the holes of vias 510 are slanted to allow covering of the walls of the holes with a conductive material without filling the holes entirely. Optionally, a conductive material is placed on the walls of the holes, forming vias 510, while the remaining volume of the holes is filled with a low x-ray absorbency material 518, which is optionally non-conductive or a poorer conductor. Alternatively, the holes are filled entirely with a conductive material.

Pads 500 are then placed on isolating layer 508. Thereafter, a filling material forming isolating strips 502 is filled in the area between pads 500. Alternatively, a layer of the material of isolating strips 502 is placed substantially on the entire surface area of probe 40, and cavities are formed therein for pads 500. Contact layer 516 is optionally firmly attached to isolating layer 508. Alternatively, contact layer 516 is manufactured separately, as a disposable contact interface which is connected to a probe base 514 which includes the remaining layers of probe 40. Thus, the advantages of using a disposable contact interface for contacting breast 33, are achieved, while, for example, using expensive materials and/or structures, which do not interfere with x-ray imaging, for lead wires 504. Optionally, pads 500 comprise suitable receptacles which fit on vias 510 of base 514. Alternatively or additionally, any other attachment method is used to connect contact layer 516 to probe base 514.

It is noted that the above manufacture method is presented by way of example and substantially any other suitable manufacture method may be used to produce probes in accordance with the present invention.

Fig. 4 is a schematic cross-section view of a portion of probe 40, in accordance with an embodiment of the present invention. Fig. 4 shows a portion of probe 40 which includes a single pad 500. In order to equalize the X-ray absorption of probe 40 over substantially its entire area which is in the path of x-ray imaging of apparatus 32, substrate 512 has different thickness at different areas, according to the absorption of the remaining elements of the probe above substrate 512. For example, probe 40 may have three different types of areas, namely, beneath pads 500, beneath vias 510 and pads 500, and not beneath pads 500. In such a probe 40, substrate 512 optionally has three respective thickness areas. Beneath via 510, substrate 512 optionally has a first thickness 610, defining a hole 620. Beneath pad 500, but not under via 510, substrate 512 optionally has a second thickness 612, defining a second hole 622, and in all other areas substrate 512 has a third thickness 614. In some embodiments of the invention, substrate 512 comprises a different material than the filling 518 of via 510 and/or pad 500. Therefore, the depth of holes 620 and/or 622 is not necessarily equal to the thickness of pad 500.

Although in some embodiments of the invention, as shown in Fig. 4, the walls of holes 620 and 622 are shown as straight lines, in other embodiments of the invention, the walls of one or more of the holes are slanted and/or rounded. The use of slanted and/or rounded holes smoothes out any artifacts which may be caused by a difference in x-ray absorption between the opposite sides of the walls of the hole.

In some embodiments of the invention, impedance probe 42 has any of the structures described above with reference to impedance probe 40. Optionally, probes 40 and 42 of an apparatus 30 have the same structure. Alternatively, probes 40 and 42 have different structures.

In some embodiments of the invention, probes 40 and 42 comprise different materials, have different layouts of lead wires 504 and/or are formed of different numbers of pieces. Optionally, probes 40 and/or 42 are marked (e.g., "upper", "lower") so that an operator can differentiate between probes 40 and 42.

In some embodiments of the invention, impedance probe 40 has a higher x-ray absorption level than probe 42. In an exemplary embodiment of the invention, the x-ray absorption level of probe 42 is allowed to be up to about 1%, while the absorption level of probe 40 is allowed to be up to about 15%. X-ray energy absorbed by either of probes 40 and 42 does not participate in formation of an image by receptor 18. On the other hand, x-ray energy absorbed by probe 40 does not impinge on breast 33, while x-ray energy absorbed by probe 42 does impinge on breast 33. Therefore, it is usually more important to minimize the x-ray absorption of probe 42 than of probe 40.

In some embodiments of the invention, impedance probe 42 has a more uniform x-ray absorption level over its surface, than probe 40. In an exemplary embodiment of the invention, the x-ray absorption differences over the area of probe 42 are less than about 0.1%, while the x-ray absorption differences over the area of probe 40 may go up to about 0.5%. This is because the scattering of x-rays within breast 33 alleviates the artifacts caused by probe 40. For example, in some embodiments of the invention, the production process of probe 42 is more accurate than of probe 40. For example, in laying alumina isolating strips 502, the allowed difference in thickness between pads 500 and strips 502 is lower than a first, low, threshold (e.g., 0.1-0.2 µm) in probe 42 and lower than a second, higher, threshold in probe 40.

In some embodiments of the invention, probe 40 comprises a single disposable piece formed of inexpensive materials which have a moderate x-ray absorbency. Probe 42, however, comprises two pieces as described above, such that probe base 514 (Fig. 3B) may comprise expensive materials which have a very low x-ray absorbency.

Alternatively or additionally, probe 40 comprises lead wires 504 with a higher conductance and higher x-ray absorbency than the lead wires 504 of probe 42. For example, the lead wires of probe 40 may have a greater cross-section area or may comprise a more conductive material. Further alternatively or additionally, the pads 500 and/or the isolating materials of strips 502 and 520, layer 508 and/or substrate 512 of probe 42 are less x-ray absorbent than the respective materials of probe 40. Further alternatively or additionally, layer 508 and/or substrate 512 are thinner in probe 42 than in probe 40.

Alternatively or additionally, probe 42 comprises a structure in accordance with any of the above described embodiments, while probe 40 has a structure known in the art, such as described in above mentioned U.S. patents 5,810,742, or 6,157,697.

In some embodiments of the invention, probes 40 and 42 have the same pad arrangements. The use of the same pad arrangements for both of probes 40 and 42 allows generating impedance images of substantially the same quality by both of probes 40 and 42 and allows more freedom in planning impedance imaging sessions.

Alternatively, probes 40 and 42 have different pad arrangements. For example, probe 42 may have a different element structure, which has a lower x-ray absorption level and/or lower x-ray absorption difference over its surface, than probe 40. In some embodiments of the invention, probe 42 has fewer pads 500 (e.g., an 8x8 array) than probe 40 (e.g., a 45x60 array) and hence requires fewer lead wires 504. Thus, thinner wires may be used for the same impedance levels on a single layer of the probe. Thus, probe 42 has less x-ray absorption differences over its area and/or less x-ray absorption. Alternatively or additionally, probe 42 is smaller than probe 40 and/or has narrower or wider isolating strips 502 between its pads 500. In some embodiments of the invention, images are normally generated based on signals sensed by probe 40. One or more images with lower resolution (due to the fewer pads 500 included in probe 42) may be provided based on signals from probe 42.

In other embodiments of the invention, one of probes 40 and 42 is used for electrification and the other is used for imaging. Using one of the probes only for electrification allows planning that probe to better suite the electrification task and/or to be less x-ray absorbent or otherwise x-ray interfering. Optionally, probe 42 comprises the electrification probe and has elongated strips of conductors arranged in parallel rows. Such elongated rows may be electrically connected from a side of probe 42 outside of the imaging region, and therefore are much less x-ray interfering. Alternatively, probe 42 comprises elements organized in circles or comprises a single planar conductive electrode.

In some embodiments of the invention, additional probes on sides of breast 33 are used in impedance imaging, in addition to probes 40 and 42. For example, two electrification probes on sides of breast 33 may be used in addition to probes 40 and 42 used for imaging on the top and bottom of breast 33. The electrification probes optionally apply electrification voltage and/or current signals from opposite sides while probes 40 and 42 sense voltage signals.

In some embodiments of the invention, one or both of probes 40 and 42 have one or more holes for inserting a needle (e.g., a biopsy needle) and/or for passing fingers of a surgeon. The probe 40 and/or 42 having the hole or holes is optionally selected as the one which will cause fewer or less troublesome artifacts on the x-ray images due to the hole. Alternatively or additionally, the holes are located on the probe which allows easier access to a physician. In some embodiments of the invention, a probe serving mainly and/or only as an electrification probe includes the holes. Thus, the area of the holes does not interfere with creation of the images.

It is noted that a wide range of impedance imaging sessions may be used with the apparatus of the present invention. Impedance imaging sessions are described, for example, in U.S. patent 5,810,742, and/or PCT/IL00/00839. Electrification signals may be applied from electrode 56, probe 40, probe 42, from other electrodes and/or from any combination of electrodes and probes. As described above, the images may be acquired by one or both of probes 40 and 42 in a single stage or in a plurality of stages.

For example, during an imaging session, some of the images are acquired by probe 42 based on electrification from probe 40 and some of the images are acquired by probe 40 based on electrification from probe 42. Alternatively or additionally, electrification signals are applied from specific elements of one or both of probes 40 and 42 and the images are acquired by the remaining elements of one or both of the probes. In an exemplary embodiment of the invention, signals are applied from one or more specific elements of probe 40 and from one or more specific elements of probe 42, while the remaining elements of probes 40 and 42 are used to generate two images from opposite sides. Optionally, the values for the pixels of the images corresponding to the elements used for electrification are calculated using a suitable interpolation method.

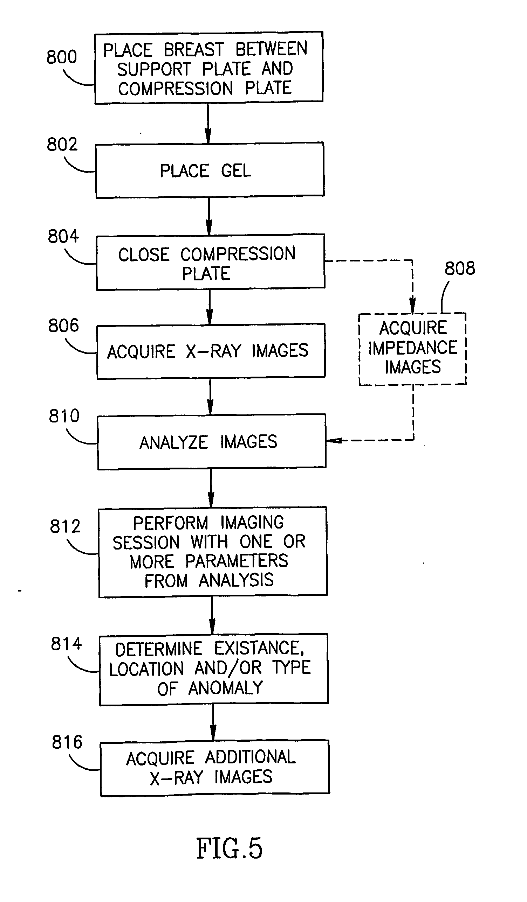

In some embodiments of the invention, a symbiotic relationship between the imaging modalities takes place. Since each technique relies on different properties of the lesion, impedance imaging enhances the understanding of x-ray imaging and vice versa. In general, coincident detection of features is expected. When anomalies are seen only on one modality, settings and methods may be somewhat altered in the other modality in order to arrive at coincident detection. Optionally, the knowledge of the improved settings and methods may be used in future imaging. An example of an imaging procedure with such a symbiotic process is now described with reference to Fig. 5.

Fig. 5 is a flowchart of the acts performed in a dual modality imaging procedure, in accordance with an embodiment of the present invention. Breast 33 is placed (800) between support plate 22 and compression plate 20. Optionally, a conductive gel layer is placed (802) between breast 33 and probe 40 and/or 42. Compression plate 22 is then closed (804) on breast 33 and one or more images are acquired (806) by x-ray tube 14 and receptor 18. Optionally, one or more impedance images are acquired (808) using impedance probes 40 and/or 42. The acquired images are analyzed (810) to detect possible anomalies in breast 33 and/or find unclear regions which require additional imaging. Responsive to the analysis, one or more additional impedance imaging sessions are performed (812) with one or more parameters selected responsive to the detected possible anomalies. The results of the additional impedance session are analyzed to determine (814) the existence, location and/or type (e.g., cancerous or non-cancerous) of the possible anomaly. Optionally, one or more additional x-ray images are acquired (816) with one or more parameters adjusted responsive to the above determination (814). The one or more adjusted parameters, may comprise for example, the angle from which the images are taken and/or the intensity of the x-rays used in the imaging, for example for unclear regions.

Referring in more detail to placing (802) the conducting gel layer, the gel may be placed by smearing the gel on the breast 33 or on probe 40, as is known in the art. Alternatively or additionally, the gel may be pre-placed on a disposable probe 40 or surface contact piece thereof, for example inside wells surrounding the pads thereof, as described for example in U.S. patent 5,810742. Further alternatively or additionally, the gel is placed in the form of an interface sheet, such as described in PCT application PCT/IL00/00287, filed May 21, 2000, and/or in PCT application PCT/IL00/00127. The use of an interface sheet isolates breast 33 from probe 40, such that a reusable probe 40 may be employed without endangering the patient. Optionally, the gel layer comprises a material with a low x-ray absorbency. Using an interface sheet also allows the use of non-bio-compatible materials for pads 500 and isolating strips 502 and therefore allows using less x-ray absorbent and/or more conductive materials. In some embodiments of the invention, the gel comprises some adhesive properties which connect probe 40 and/or 42 to the breast.

Referring in more detail to analyzing (810) the x-ray images and optionally impedance images, in some embodiments of the invention, the analysis is performed automatically by a processor, e.g., computer 24. Alternatively or additionally, the analysis is performed by a human operator. For example, the images may be displayed on monitor 26 for examination by the operator. In some embodiments, both the x-ray apparatus and the impedance probe produce digital images that are displayed on monitor 26. The images may be displayed one after the other, one next to the other and/or overlaid one on top of the other. Alternatively, film images of the two modalities are produced and superimposed on a light box. Alternatively, the impedance image is printed on a transparency for viewing with a film image, on a light box. Alternatively still, a film image is scanned in order to produce a digital image, and the digital images are overlaid and viewed on a computer monitor.

In some embodiments of the invention, digital images that are overlaid and viewed on the computer monitor can undergo image processing by the computer. Image processing may comprise changing the dimensions of one image, in order to match the dimensions of the other image; zooming in on an area, removing noise of any kind and other image-enhancement processing as known in the art.

Optionally, x-ray images acquired while probes 40 and/or 42 are in place, are processed by a signal processing program, running for example on computer 24, which removes the anticipated pattern of multi-element impedance probe 40. Optionally, computer 24 identifies the artifacts on the acquired x-ray images and removes them accordingly. Alternatively or additionally, computer 24 removes shadowing artifacts expected for the layout of probes 40 and/or 42. Optionally, computer 24 stores one or more known probe layouts and uses for post processing the stored layout which belongs to the probes 40 and/or 42 currently used. Alternatively or additionally, before an imaging session, a blank image is taken with probes 40 and/or 42 in place but without breast 33, and the blank image is analyzed to determine the artifacts caused by the probes.

In some embodiments of the invention, the results of the analysis (810) determine the depth of a suspected anomaly and the additional analysis (814) is used to determine the type of the anomaly, e.g., whether the anomaly has the characteristics of a malignant region. The depth may be determined by acquiring x-ray images from two different angles, as is known in the art. Alternatively or additionally, the analysis (810) is used to determine a two dimensional location of an anomaly and the additional analysis (814) is used to determine the depth of the anomaly. Further alternatively or additionally, the results of the analysis (810) indicate an unclear area and the additional analysis (814) is used to carefully scan the unclear area.

Further alternatively or additionally, the results of the analysis (810) are used in normalizing the results of the impedance imaging. Optionally, the analysis of the x-ray image and/or the combined image determine an area with a suspected area and a clear area most distinctly without anomalies. The results of the impedance imaging (812) of the clear area are used to normalize the results of the imaging of the suspected area, thus providing a better view of the suspected anomaly and/or more accurate data on the anomaly.

In an exemplary embodiment of the invention, one of probes 40 and 42 is used for sensing signals used in forming an image, while the other probe is used to provide electrification signals to breast 33. Optionally, the additional impedance imaging session is performed with specific electrification patterns and/or sensing patterns suitable for examining the suspected anomaly. For example, the electrification signals may be applied to two parallel lines of pads on opposite sides of the suspected anomaly, as described in PCT application PCT/IL00/00839. In some embodiments of the invention, the distance between parallel lines and a point above the suspected anomaly is substantially equal to the depth of the anomaly. Optionally, the electrification signals to the parallel lines have different phases, optionally opposite phases.

Alternatively or additionally, any other impedance imaging methods, such as described, for example, in PCT/IL00/00839 and/or in U.S. patent 5,810742, are used. Any of the parameters of such imaging methods may be adjusted responsive to the analysis (810). In some embodiments of the invention, the adjusted parameters comprise the amplitude and/or frequency of the electrification signals. For example, deeper anomalies may require electrification signals with a higher amplitude and/or a different frequency than used for shallow anomalies. Alternatively or additionally, the adjusted parameters comprise a determination of which pads of the electrification probe are used in the electrification, e.g., how many of the pads and/or in which configuration. Further alternatively or additionally, the adjusted parameters comprise a determination of which elements (on one probe or two probes) are used for electrification and which elements are used for sensing. Further alternatively or additionally, the adjusted parameters comprise a determination of whether a remote electrode is to be used.

In some embodiments of the invention, in which the applied signals have different phases as described in PCT/IL00/00839, the adjusted parameter comprises the phase difference between the applied signals.

In some embodiments of the invention, after determining the location and/or type of the anomaly, a biopsy needle is directed to the anomaly in order to receive additional information on the anomaly. The guidance of the needle toward the anomaly may be performed using impedance imaging methods, such as described in PCT/IL00/00839 and/or U.S. patent 5,810,742. Optionally, an electrical signal is applied to the biopsy needle so that its advance is easily detected by probe 40 and/or 42. It is noted that the guidance using impedance imaging is possible even for anomalies which are only detected based on x-ray images, based on the registration of the impedance images and the x-ray images. An additional x-ray image may be taken when the biopsy needle reaches the designated point as an additional verification that the needle is at the correct point.

Referring in more detail to acquiring (816) the additional x-ray images, the additional images are acquired from different angles and/or with different x-ray beam intensities. The different angles and/or intensities are optionally selected responsive to an analysis of previously acquired images. For example, additional x-ray images may be acquired from an angle in which probes 40 and/or 42 cause fewer artifacts in a specific area of interest.

It is noted that the entire imaging process may be repeated for a different angular view of breast 33, for example, a lateral view, in which the apparatus is rotated, and the breast is compressed from the side.

In some embodiments, the symbiotic relationship is further extended to the understanding of what features and types of lesions are better visualized by x-ray, and what features and what types of lesions are better detected by impedance. As such, specificity and sensitivity of each technique to certain features and types of lesions may be studied and compared. Since different sensitivities exist, the use of both modalities together allows for more thorough diagnosis.

Alternatively or additionally to adjusting the acquisition of data of one modality responsive to imaging results from another modality, the display of the data acquired for one of the modalities is adjusted responsive to results from another modality.

In some embodiments of the invention, the type of the parameter of the impedance imaging which is displayed (e.g., critical frequency, phase, capacitance, conductivity) is selected responsive to results from the imaging of another modality. Alternatively or additionally, during the impedance imaging, data is collected for a plurality of stimulation signals and the data displayed is chosen responsive to the imaging results from another modality. For example, data may be acquired with applied stimulation signals at a plurality of locations, layouts, phase differences, frequencies and/or amplitudes. The displayed image is optionally chosen responsive to data from images of another modality, e.g., the location of a suspected anomaly in acquired x-ray images. For example, for a suspected anomaly at a depth x beneath probe 40, data may be displayed from imaging performed with stimulation signals applied from opposite sides, optionally at distance x, from the surface area above the suspected anomaly.

In some embodiments of the invention, combined mammography and impedance imaging procedures are performed for a plurality of patients one after the other using the same apparatus. For safety purposes, before the breast of each patient is placed in the mammogram, a disposable contact surface is placed on compression plate 20 and/or on support plate 22. The contact surface may comprise, as described above, a gel sheet a probe 40 and/or 42 and or a contact layer of a probe, for example including pads 500 without leading wires 502. Changing the contact surface between patients eliminates the need for sterilization and/or cleaning of the apparatus between imaging procedures.

Alternatively or additionally to the method of Fig. 5, any other combined impedance imaging and x-ray imaging method may be used. Particularly, the x-ray images and impedance images may be obtained in any order and/or simultaneously.

The method of Fig. 5 and/or other imaging methods in which the parameters of one modality are adjusted responsive to results of imaging using another modality, are not limited to the above described apparatus, but rather may be used with substantially any x-ray apparatus known in the art, including apparatus for imaging tissue other than the breast.

In some embodiments of the invention, instead of having all of probe 42 in place during the acquisition of the x-ray image, only a body contact portion of probe 42 is in place. After the x-ray image is acquired, the remaining parts of probe 42 are put in place, optionally after removing receptor 18. Optionally, the body contact portion comprises a thick non-conductive substrate of a very low x-ray absorbance, which can serve as a support plate. The contact portion includes a surface facing the breast, pads 500 as described above. Thin conductive vias lead, through the substrate, from pads 500 to an opposite side of the substrate. A second part of probe 42 includes leading wires 504 and ports which connect the vias from the body contact portion to the leading wires.

The principles of the present invention are not limited to dual modality apparatus. In some embodiments of the invention, an additional modality, beyond impedance and X-ray, is used in examining breast 33. The third modality may be substantially any suitable imaging method known in the art, such as gamma imaging. For example, a gamma camera may fit onto impedance probe 40 of compression plate 20.

It will be appreciated that the above described methods may be varied in many ways, including, changing the order of steps, and/or performing a plurality of steps concurrently. For example, the steps described above of manufacturing probe 40 may be performed in a different order. It should also be appreciated that the above described description of methods and apparatus are to be interpreted as including apparatus for carrying out the methods, and methods of using the apparatus. The present invention has been described using non-limiting detailed descriptions of embodiments thereof that are provided by way of example and are not intended to limit the scope of the invention. It should be understood that features and/or steps described with respect to one embodiment may be used with other embodiments and that not all embodiments of the invention have all of the features and/or steps shown in a particular figure or described with respect to one of the embodiments. Variations of embodiments described will occur to persons of the art. Furthermore, the terms "comprise," "include," "have" and their conjugates, shall mean, when used in the claims, "including but not necessarily limited to."

It is noted that some of the above described embodiments may describe the best mode contemplated by the inventors and therefore may include structure, acts or details of structures and acts that may not be essential to the invention and which are described as examples. Structure and acts described herein are replaceable by equivalents which perform the same function, even if the structure or acts are different, as known in the art. Therefore, the scope of the invention is limited only by the elements and limitations as used in the claims.

## Claims

1. A probe for impedance imaging comprising:
a plurality of conductive elements (500) adapted to contact tissue of a subject;
a plurality of conductive wires (504) connecting the conductive elements (500) to an external connector (506); and
one or more non-conductive materials (502, 508, 512, 520),
**characterized in that**
wherein the x-ray absorbance of the non-conductive materials (502, 508, 512, 520), in at least some of the surface areas of the probe, is a function of the x-ray absorbance of the conductive wires (504) and elements (500) in the same surface area, such that the probe has substantially the same x-ray absorbance over most of the surface area of the probe.

2. A probe according to claim 1, wherein the one or more non-conductive materials (502, 508, 512, 520) comprise a substrate layer (512) having different thickness at different surface areas of the probe.

3. A probe according to claim 2, wherein the substrate layer (512) is thinner in surface areas of the probe which include conductive wires (504).

4. A probe according to any of the preceding claims, wherein the one or more non-conductive materials (502, 508, 512, 520) comprise at least one isolating material which has an x-ray absorbency substantially equal to the x-ray absorbency of the conductive elements (500) or of the conductive wires (504).

5. A probe according to claim 4, wherein the plurality of conductive elements (500) comprise an aluminum-based deposit and the at least one isolating material (502, 508, 512, 520) comprises alumina.

6. A probe according to any of the preceding claims, wherein the one or more non-conductive materials (502, 508, 512, 520) comprise at least one isolating material (502) which is deposited between at least some of the conductive elements (500).

7. A probe according to any of the preceding claims, wherein the x-ray absorption level of at least some of the surface areas including the conductive elements (500) is substantially equal to the absorption level of at least some of the surface areas not including the conductive elements (500).

8. A probe according to any of the preceding claims, wherein the plurality of conductive wires (504) are included in a single layer, which layer has a substantially equal x-ray absorption level over substantially its entire surface area.

9. A probe according to any of the preceding claims, wherein the probe does not include conductive portions which are not included in an electrical path between one of the conductive elements (500) and the external connector (506).

10. A probe according to any of the preceding claims, wherein the probe has an x-ray absorption level of less than 6%.

## Patentansprüche

1. Sonde zur Impedanzabbildung, umfassend:
eine Mehrzahl von leitenden Elementen (500), die angepasst sind, um mit einem Gewebe eines Subjekts Kontakt zu machen;
eine Mehrzahl von leitenden Drähten (504), die die leitenden Elemente (500) mit einem externen Verbinder (506) verbinden; und
ein oder mehrere nichtleitende Materialien (502, 508, 512, 520),
**dadurch gekennzeichnet, dass**
die Röntgenstrahlextinktion der nichtleitenden Materialien (502, 508, 512, 520) in mindestens einigen der Oberflächenbereiche der Sonde eine Funktion der Röntgenstrahlextinktion der leitenden Drähte (504) und Elemente (500) in demselben Oberflächenbereich ist, so dass die Sonde im Wesentlichen dieselbe Röntgenstrahlextinktion über den größten Teil des Oberflächenbereichs der Sonde aufweist.

2. Sonde nach Anspruch 1, bei der das eine oder die mehreren nichtleitenden Materialien (502, 508, 512, 520) eine Substratschicht (512) mit unterschiedlicher Dicke bei unterschiedlichen Oberflächenbereichen der Sonde umfassen.

3. Sonde nach Anspruch 2, bei der die Substratschicht (512) in Oberflächenbereichen der Sonde dünner ist, die leitende Drähte (504) umfassen.

4. Sonde nach einem der vorangehenden Ansprüche, bei der das eine oder die mehreren nichtleitenden Materialien (502, 508, 512, 520) mindestens ein Isoliermaterial umfassen, das eine Röntgenstrahlextinktion aufweist, die im Wesentlichen gleich der Röntgenstrahlextinktion der leitenden Elemente (500) oder der leitenden Drähte (504) ist.

5. Sonde nach Anspruch 4, bei der die Mehrzahl von leitenden Elementen (500) ein Ablagerung auf Aluminium-Basis umfassen und das mindestens eine Isoliermaterial (502, 508, 512, 520) Aluminiumoxid umfasst.

6. Sonde nach einem der vorangehenden Ansprüche, bei der das eine oder die mehreren nichtleitenden Materialien (502, 508, 512, 520) mindestens ein Isoliermaterial (502) umfassen, das zwischen mindestens einigen der leitenden Elemente (500) abgelagert ist.

7. Sonde nach einem der vorangehenden Ansprüche, bei der das Röntgenstrahlabsorptionsniveau von mindestens einigen der Oberflächenbereiche, die die leitenden Elemente (500) umfassen, im Wesentlichen gleich dem Adsorptionsniveau von mindestens einigen der Oberflächenbereiche ist, die die leitenden Elemente (500) nicht umfassen.

8. Sonde nach einem der vorangehenden Ansprüche, bei der die Mehrzahl von leitenden Drähten (504) in einer einzigen Schicht enthalten sind, welche Schicht ein im Wesentlichen gleiches Röntgenstrahlabsorptionsniveau über im Wesentlichen ihrem ganzen Oberflächenbereich aufweist.

9. Sonde nach einem der vorangehenden Ansprüche, bei der die Sonde keine leitenden Teile enthält, die nicht in einem elektrischen Pfad zwischen einem der leitenden Elemente (500) und dem externen Verbinder (506) enthalten sind.

10. Sonde nach einem der vorangehenden Ansprüche, bei der die Sonde ein Röntgenstrahlabsorptionsniveau von weniger als 6% aufweist.

## Revendications

1. Sonde pour imagerie d'impédance, comportant :
une pluralité d'éléments conducteurs (500) adaptés pour venir au contact d'un tissu d'un sujet,
une pluralité de fils conducteurs (504) reliant les éléments conducteurs (500) à une connecteur externe (506), et
un ou plusieurs matériaux non-conducteurs (502, 508, 512, 520),
**caractérisée en ce que**
l'absorbance des rayons X des matériaux non-conducteurs (502, 508, 512, 520), dans au moins une partie des zones de surface de la sonde, est fonction de l'absorbance des rayons X des fils conducteurs (504) et des éléments (500) dans la même zone de surface, de sorte que la sonde a sensiblement la même absorbance des rayons X sur la majeure partie de la zone de surface de la sonde.

2. Sonde selon la revendication 1, dans laquelle les un ou plusieurs matériaux non-conducteurs (502, 508, 512, 520) comportent une couche de substrat (512) ayant une épaisseur différente dans des zones de surface différentes de la sonde.

3. Sonde selon la revendication 2, dans laquelle la couche de substrat (512) est plus mince dans les zones de surface de la sonde qui incluent des fils conducteurs (504).

4. Sonde selon l'une quelconque des revendications précédentes, dans laquelle les un ou plusieurs matériaux non-conducteurs (502, 508, 512, 520) comportent au moins un matériau isolant qui a une absorbance des rayons X sensiblement égale à l'absorbance des rayons X des éléments conducteurs (500) ou des fils conducteurs (504).

5. Sonde selon la revendication 4, dans laquelle la pluralité d'éléments conducteurs (500) comportent un dépôt à base d'aluminium et le au moins un matériau isolant (502, 508, 512, 520) comporte de l'alumine.

6. Sonde selon l'une quelconque des revendications précédentes, dans laquelle les un ou plusieurs matériaux non-conducteurs (502, 508, 512, 520) comportent au moins un matériau isolant (502) qui est déposé entre au moins une partie des éléments conducteurs (500).

7. Sonde selon l'une quelconque des revendications précédentes, dans laquelle le niveau d'absorption des rayons X d'au moins une partie des zones de surface incluant les éléments conducteurs (500) est sensiblement égal au niveau d'absorption d'au moins une partie des zones de surface n'incluant pas les éléments conducteurs (500).

8. Sonde selon l'une quelconque des revendications précédentes, dans laquelle la pluralité de fils conducteurs (504) sont incorporés dans une couche unique, laquelle couche a un niveau d'absorption des rayons X sensiblement égal sur sensiblement toute sa zone de surface.

9. Sonde selon l'une quelconque des revendications précédentes, dans laquelle la sonde n'inclut pas de parties conductrices qui ne sont pas incluses dans un trajet électrique entre un des éléments conducteurs (500) et le connecteur externe (506).

10. Sonde selon l'une quelconque des revendications précédentes, dans laquelle la sonde a un niveau d'absorption des rayons X inférieur à 6 %.
